# FASCICULE DE BREVET EUROPEEN

(11) **EP 2 253 052 B1**
(45) Date de publication et mention de la délivrance du brevet: **29.08.2012**
(21) Numéro de dépôt: 08872605.4
(22) Date de dépôt: 09.12.2008
(51) Int. Cl.: H01R 24/20, A61B 5/042, A61B 5/0402

(54) **DISPOSITIF DE CONNEXION ET SYSTÈME MÉDICAL D'ACQUISITION DE SIGNAUX ÉLECTRIQUES ÉQUIPÉ D'UN TEL DISPOSITIF DE CONNEXION**
ANSCHLUSSVORRICHTUNG UND MEDIZINISCHES ELEKTRISCHES SIGNALERFASSUNGSSYSTEM MIT EINER DERARTIGEN ANSCHLUSSVORRICHTUNG
CONNECTION DEVICE AND MEDICAL ELECTRICAL SIGNALS COLLECTION SYSTEM USING SUCH A CONNECTION DEVICE

(30) Priorité: 10.12.2007 FR 0708574
(43) Date de publication de la demande: 24.11.2010
(73) Titulaire: Integral Process, 78700 Conflans-Sainte-Honorine (FR)
(72) Inventeur: MONIER, Hubert, F-78700 conflans-Sainte-Honorine (FR)
(74) Mandataire: Broydé, Marc
(86) Numéro de dépôt international: PCT/FR2008/001713
(87) Numéro de publication internationale: WO 2009/103873

(56) Documents cités:
- EP-A- 1 195 855
- WO-A-01/76019
- WO-A-99/23729
- FR-A- 1 416 048
- US-A- 5 147 215
- US-A- 5 622 168
- US-B1- 6 786 775

## Description

L'invention concerne le domaine de la connectique pour la transmission de signaux électriques.

Plus particulièrement, l'invention concerne un connecteur femelle, un connecteur mâle, un dispositif de connexion pour la transmission de signaux électriques comportant de tels connecteurs, femelle et mâle, et un système médical d'acquisition de signaux électriques, équipé d'un tel dispositif de connexion.

Dans l'art antérieur, de nombreux types de connecteur sont connus. Parmi cette diversité, les connecteurs RJ, Registered Jack ou prise jack enregistrée, constituent des standards internationaux utilisés notamment pour les appareils téléphoniques fixes ou dans le domaine des réseaux informatiques.

Les connecteurs RJ se composent d'un connecteur femelle et d'un connecteur mâle. Le connecteur femelle comprend un boîtier délimitant une cavité de réception d'un connecteur mâle et des contacts, destinés à coopérer avec les contacts correspondants du connecteur mâle, s'étendant le long d'une des parois de la cavité. Le connecteur mâle comprend un corps en matériau diélectrique, comportant un logement pourvu d'une ouverture pour le passage de câbles et des griffes de contact auto dénudantes insérées dans des encoches, formées sur une des faces extérieures du corps.

Ce type de connecteur est peu coûteux, facile à fabriquer et peut être raccordé facilement aux câbles transmettant les signaux électriques.

Toutefois, l'isolation électrique entre les contacts de ces connecteurs est fortement limitée. Ainsi, lorsque les signaux électriques présentent de fortes intensités, les risques de formation d'un arc électrique entre deux contacts des connecteurs sont élevés.

Ainsi, ces connecteurs ne peuvent pas être utilisés dans certains domaines d'application.

C'est notamment le cas dans le domaine des systèmes médicaux d'acquisition de signaux électriques, tels que, par exemple, les systèmes pour la réalisation d'électrocardiogrammes, qui ne peuvent pas utiliser des connecteurs du type RJ.

En effet, dans une telle application, les connecteurs sont raccordés à des câbles associés à des électrodes de surveillance ECG placées près du coeur du patient. Ainsi, il est possible d'obtenir des signaux permettant d'étudier les pathologies cardiovasculaires. Pour certaines pathologies, une forte décharge électrique, de défibrillation, est administrée en urgence au patient, à proximité du coeur. Or, si la décharge électrique est administrée lorsque les électrodes sont placées sur la peau près du coeur du patient, il existe un risque important de formation d'un arc électrique entre les contacts du connecteur, si ceux-ci ne sont pas suffisamment isolés électriquement. La formation d'un arc électrique est à proscrire totalement car un tel phénomène risque d'endommager le dispositif d'acquisition des signaux d'électrocardiographie, qui ne permettrait plus alors de suivre les évolutions cardiaques ; et de créer des perturbations électromagnétiques nuisibles. En outre, ce phénomène absorbe une partie de l'énergie de la décharge électrique et diminue ainsi l'efficacité de la défibrillation au patient.

Par conséquent, les connecteurs du type RJ ne peuvent pas être utilisés pour ce type d'application et il est d'usage d'utiliser des connecteurs à broches comprenant un connecteur mâle comportant une pluralité de broches de contacts et un connecteur femelle comprenant une pluralité d'alvéoles de réception des broches, séparées par un matériau diélectrique.

Toutefois, ces connecteurs sont relativement coûteux et complexes à fabriquer. En outre, le raccordement des connecteurs aux câbles est le plus souvent réalisé par soudure, ce qui augmente d'autant plus les coûts de mise en oeuvre.

US 6 786 775 B1 décrit un dispositif de connexion pour la transmission de signaux électriques comportant un connecteur RJ femelle et un connecteur RJ mâle selon le préambule de la revendication 1.

L'invention vise à remédier aux problèmes de l'art antérieur mentionnés ci-dessus, en proposant un dispositif de connexion qui soit, à la fois simple, peu coûteux et présente une bonne isolation électrique entre les contacts.

À cet effet, l'invention propose, selon un premier aspect, un connecteur femelle pour la transmission de signaux électriques comprenant :
- un boîtier comportant des parois délimitant une cavité de réception d'un connecteur mâle ;
- des premiers doigts de contacts, s'étendant parallèlement à une première paroi et destinés à coopérer avec des contacts correspondants du connecteur mâle ; et
- des cloisons d'isolation en matériau diélectrique faisant saillie de la première paroi vers l'intérieur de la cavité et disposées entre les premiers doigts de contact.

Ainsi, la fabrication du connecteur femelle est simple et peu coûteuse et l'isolation électrique est nettement améliorée par les cloisons d'isolation.

Avantageusement, le connecteur comprend des seconds doigts de contact s'étendant parallèlement à une seconde paroi, opposée à la première paroi, et des cloisons d'isolation, en matériau diélectrique, faisant saillie de la seconde paroi vers l'intérieur de la cavité et disposées entre les seconds doigts de contact. Les doigts de contact sont donc répartis le long d'au moins deux parois opposées. Ainsi, selon l'invention, deux parois sont utilisées pour la disposition des doigts, ce qui permet d'accroître les distances entre les doigts de contact et d'augmenter ainsi l'isolation.

Avantageusement, les premiers et les seconds doigts de contact sont disposés, en quinconce, les uns par rapport aux autres. Ainsi, les doigts de contact sont régulièrement répartis, de part et d'autre du corps du connecteur mâle.

Les cloisons d'isolation peuvent notamment être réalisées en polyamide qui est un matériau présentant un très bon compromis entre ses caractéristiques d'isolation et son coût.

Les doigts de contacts peuvent être en laiton. Le laiton est à la fois avantageux et résistant mécaniquement à un grand nombre de connexions.

Dans un mode de réalisation, les doigts de contact présentent une section cylindrique.

Avantageusement, au moins une des parois du boîtier est équipée d'au moins un repère détrompeur. Ainsi, les risques d'utilisations non appropriées du connecteur sont réduits.

Avantageusement, au moins une des parois est équipée de moyens de butée permettant de maintenir le connecteur mâle dans la cavité.

Selon un second aspect, l'invention propose un connecteur mâle pour la transmission de signaux électriques comprenant :
- un corps en matériau diélectrique, destiné à être introduit dans un connecteur femelle selon le premier aspect de l'invention, comprenant des doigts de contact et des cloisons d'isolation, ledit corps comportant :
   - un logement pourvu d'une ouverture pour le passage de câbles ;
   - des faces extérieures destinées à venir en vis-à-vis des parois délimitant la cavité du connecteur femelle ;
   - des encoches formées sur au moins une première face extérieure munies d'une lumière débouchant dans ledit logement ; et
- des griffes de contacts, insérées dans lesdites lumières, comportant une piste de contact destinée à coopérer avec les doigts de contact du connecteur femelle et une portion auto dénudante s'étendant dans le logement ;
chaque encoche présente des dimensions adaptées pour recevoir un doigt de contact et une des cloisons d'isolation de manière à constituer une séparation entre ladite encoche et une encoche voisine.

De même, le connecteur est simple à fabriquer et le raccordement des câbles au connecteur mâle est facilement réalisé au moyen des griffes auto dénudantes de contact. En outre, pour chaque encoche, le doigt et la griffe de contact, respectivement du connecteur femelle et mâle, sont isolés des autres contacts par la cloison d'isolation du connecteur femelle et la barrière délimitant l'encoche qui forment ensemble un obstacle isolant.

Avantageusement, chaque encoche présente, entre la lumière et un bord de ladite encoche, un premier espace de réception d'un doigt de contact ou d'une cloison d'isolation. En outre, chaque encoche présente, entre la griffe de contact et le plan de la première paroi, un second espace de réception d'une cloison d'isolation ou d'un doigt de contact.

Avantageusement, des encoches munies d'une lumière débouchant dans le logement sont formées sur une seconde face extérieure, opposée à la première face extérieure ; des griffes de contacts étant insérées dans lesdites lumières. En outre, les griffes de contact insérées dans les lumières de la première face extérieure et celles de la seconde face extérieure sont disposées en quinconce. Ainsi, les contacts sont régulièrement répartis et la distance les séparant est accrue.

Avantageusement, la portion auto dénudante présente une forme en W, s'étendant dans le plan longitudinal du connecteur et dont les deux branches sont destinées à sectionner la gaine du câble. Ainsi, une telle griffe de contact permet d'obtenir une bonne qualité de connexion.

Selon un troisième aspect, l'invention concerne un dispositif de connexion pour la transmission de signaux électriques comprenant un connecteur femelle selon le premier aspect de l'invention et un connecteur mâle selon le second aspect de l'invention.

Enfin, selon un quatrième aspect, l'invention propose un système médical d'acquisition de signaux électriques comprenant :
- des électrodes destinées à être mise en contact avec le corps d'un patient ;
- un dispositif de connexion à des moyens de traitement des signaux électriques ;
- des câbles connectés d'une part auxdites électrodes et d'autre part au dispositif de connexion ;
- le dispositif de connexion est un dispositif de connexion selon un troisième aspect de l'invention.

Ainsi, en raison de la facilité de fabrication du connecteur, le système médical selon l'invention présente de faibles coûts de fabrication et peut notamment être à usage unique. Par conséquent, un tel système médical permet de diminuer les risques sanitaires de maladies infectieuses liés à l'utilisation de ce système et/ou de s'affranchir des opérations de stérilisation.

En outre, un tel système peut être utilisé simultanément à l'administration d'un choc de défibrillation.

D'autres objets et avantages de l'invention apparaîtront au cours de la description qui suit, faite en référence aux dessins annexés, dans lesquels :
- la figure 1 est une vue en perspective d'un connecteur mâle selon l'invention ;
- la figure 2 est une vue en perspective, en coupe longitudinale du connecteur mâle de la figure 1 ;
- la figure 3 est une vue de dessus du connecteur mâle de la figure 1 ;
- la figure 4 est une vue arrière du connecteur mâle de la figure 1 ;
- la figure 5 représente une griffe de contact du connecteur mâle ;
- la figure 6 est une vue en perspective illustrant un connecteur mâle et un connecteur femelle, selon l'invention, lors de l'introduction du connecteur mâle dans le connecteur femelle ;
- la figure 7 est une vue en perspective d'un connecteur femelle selon l'invention ;
- la figure 8 est une vue arrière du boîtier du connecteur femelle de la figure 7;
- la figure 9 est une vue de côté d'un doigt de contact du connecteur femelle ;
- la figure 10 est une représentation schématique d'un système médical d'acquisition des signaux électriques provenant du corps humain, selon l'invention ; et
- la figure 11 est une représentation détaillée, en coupe transversale, d'un dispositif de connexion selon l'invention comprenant un connecteur mâle inséré dans le connecteur femelle.

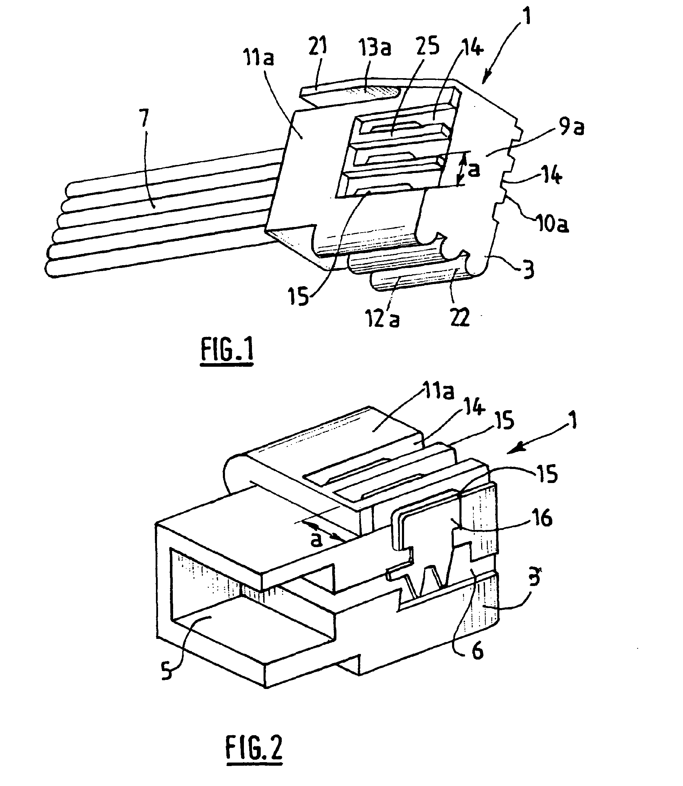

Le connecteur mâle 1, illustré sur les figures 1 à 4, comprend un corps 3 en matériau diélectrique destiné à être introduit dans la cavité 4 du connecteur femelle 2. Le corps 3 est une pièce en plastique moulée et peut notamment être réalisé en polycarbonate.

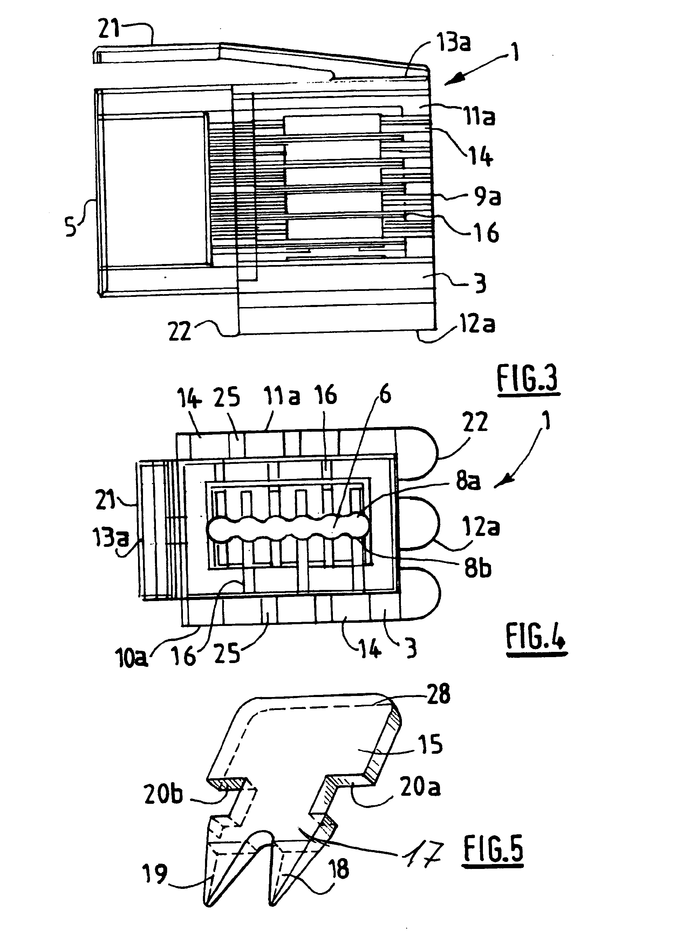

En face arrière, le corps 3 présente une ouverture 5 débouchant sur un logement 6, permettant l'introduction des câbles 7 à l'intérieur dudit corps 3. Avantageusement, afin d'assurer le guidage des câbles 7 à l'intérieur du logement 6, le corps 3 est muni de gorges 8a, 8b de guidage des câbles 7, représentées de façon détaillée sur la figure 4. Les gorges 8a, 8b sont formées sur les parois intérieures, supérieure et inférieure, du logement 6 et présentent un profil en arc de cercle, dont le rayon est légèrement supérieur au diamètre des câbles 7.

Le corps 3 comporte cinq faces extérieures destinées à venir en vis-à-vis des parois de la cavité 4 du connecteur femelle 2: une face avant 9a, une face droite 12a, une face gauche 13a, une face supérieure 11a et une face inférieure 10a.

Des encoches 14 de réception des griffes de contact 15 sont formées dans les faces supérieure 11a et inférieure 10a du corps 3. Les encoches 14 sont séparées par des barrières isolantes 25, formées dans lesdites faces supérieure 11a et inférieure 10a. Avantageusement, les encoches 14 présentent une hauteur, supérieure à 0,8 mm, de préférence supérieure ou égale à 1 mm, et une largeur, supérieure à 1 mm, de préférence supérieure ou égale à 1,4 mm. En outre, les barrières isolantes 25 présentent une épaisseur, supérieure à 0,5 mm, de préférence supérieure ou égale à 0,6 mm.

Des lumières 16 débouchant à l'intérieur du logement sont formées dans les encoches 14. Les lumières 16 permettent l'insertion des griffes de contact 15 à l'intérieur du corps 3.

Les griffes de contact 15, représentées sur la figure 5, comportent une piste de contact 28 qui émerge du boîtier 3 et une portion auto dénudantes 17 insérée à l'intérieur du logement 6. La portion auto dénudante 17 présente une forme en W, qui s'étend dans le plan longitudinal du connecteur 1. Ainsi, les deux branches 18, 19 du W sectionnent la gaine du câble 7, en deux points espacés le long de chaque câble 7. Les branches 18, 19 permettent de réaliser la connexion électrique et permettent en outre, de maintenir les câbles 7 dans le corps 3. Les griffes de contact 15 possèdent deux épaulements 20a, 20b venant en butée contre deux surfaces d'appui, formées à l'intérieur des lumières 16. Dans un mode de réalisation, les griffes de contact 15 peuvent être réalisées dans un matériau conducteur, transparent aux rayons X, tel que le carbone alors que, dans un autre mode de réalisation, celles-ci sont réalisées en laiton.

Les griffes de contact 15, ainsi que les encoches 14 et lumières 16 sont disposées, en quinconce, de part et d'autre du corps 3 du connecteur 1. Les griffes de contact 15 sont disposées parallèlement les une aux autres et sont alternativement dirigées vers la face supérieure 11a et vers la face inférieure 10a. Ainsi, les distances « a » séparant les pistes des contacts, disposées sur la même face 10a, 11a du corps 3 sont accrues, ce qui permet d'augmenter l'isolation entre les griffes de contact 15.

En outre, les portions auto dénunantes 17 des griffes 15 s'enfoncent alternativement par le dessus et par le dessous des câbles 7. Ainsi, les portions auto dénudantes 17 de deux griffes voisines 15 sont isolées par les gaines isolantes des câbles 7. En outre, les câbles 7 étant maintenues dans des gorges 8a, 8b dont les dimensions ne sont que légèrement supérieures à celles des câbles 7, le corps 3 assure également une isolation électrique entre les griffes 15, au niveau du logement 6.

Le raccordement des câbles 7 au connecteur mâle 1 est opéré de la manière suivante. Dans un premier temps, les câbles 7 sont insérés à l'intérieur du logement 6 via l'ouverture 5 et placés en butée contre la paroi intérieure avant du logement 6. Par la suite, les griffes de contacts 15 sont poussées à l'intérieur du logement 6, au moyen d'une pince à sertir par exemple, jusqu'à ce que les épaulements 20a, 20b viennent en butée contre les surfaces d'appui formées dans les lumières 16. Ainsi, les branches 18, 19 sont connectées aux câbles 7 en perçant la gaine isolante. Par conséquent, il n'est pas nécessaire de dénuder les fils 7 avant de les introduire dans le logement 6 du connecteur mâle 1.

Le connecteur 1 représenté permet la connexion de six câbles. Ainsi, dans le mode de réalisation représenté, trois encoches 14 sont formées dans la face supérieure 11 a et trois encoches 14, chacune décalées latéralement par rapport aux encoches 14 de la face supérieure 11a, sont formées dans la face inférieure 10a.

Par ailleurs, la face latérale gauche 13a est équipée des moyens de verrouillage comprenant une languette élastique 21 de verrouillage et une surface de butée portée par la languette élastique 21. La surface de butée vient coopérer avec des moyens de butée formés sur le connecteur femelle, de façon à maintenir le connecteur mâle 1, en place dans la cavité 4.

La face latérale droite 12a est équipée d'un repère détrompeur 22 qui est, dans le mode de réalisation représenté, composé de trois demi-cylindres destinés à venir s'insérer dans des formes complémentaires 29 formées dans une paroi 12b du boîtier 23 du connecteur femelle 2.

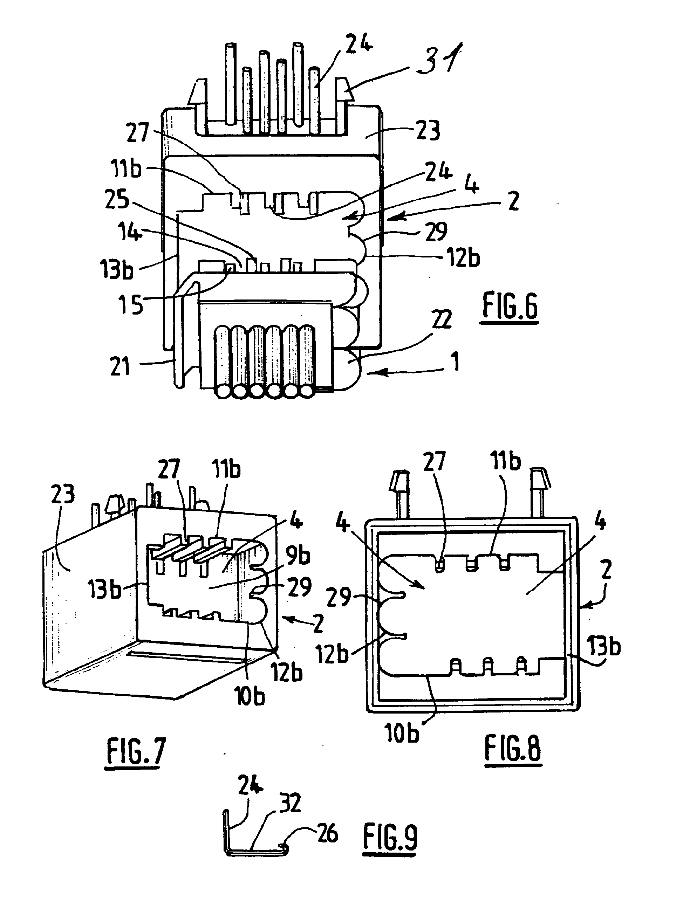

Le connecteur femelle 2, illustrée sur la figure 7 et 8, comprend un boîtier 23 pourvu d'une cavité 4 délimitée par cinq parois : gauche 13b, droite 12b, supérieure 11 b, inférieure 10b et une paroi de fond 9b. Le boîtier 23 est également une pièce plastique moulée dans un matériau diélectrique, pouvant notamment être réalisée en polyamide.

Des doigts de contact 24, illustrés sur la figure 9, comprennent une portion de contact 32 qui s'étend le long des parois, supérieure 11b et inférieure 10b, parallèlement à celles-ci. La portion de contact 32 comprend une extrémité recourbée 26 venant en contact contre les parois 10b et 11b et assurant une élasticité à la portion de contact 32. Par ailleurs, les doigts de contact 24 sont, de préférence, réalisés en laiton.

Les positions des doigts de contacts 24 sur les parois, supérieure 11b et inférieure 10b, sont agencées de manière à ce que les pistes des dits doigts de contact 24 coopèrent avec les griffes de contacts 15 correspondantes du connecteur mâle 1. Ainsi, les doigts de contact 24 sont également disposés en quinconce. Dans le mode de réalisation représenté, les doigts de contacts 24 d'une même paroi 10b, 11b sont éloignés d'une distance supérieure à 1 mm, de préférence supérieure ou égale à 1,4 mm.

Des cloisons d'isolation 27, formées dans les parois supérieure 11b et inférieure 10b sont disposées entre les doigts de contact 24. Les cloisons d'isolation 27 font saillies des parois 11 b, 10b vers l'intérieur de la cavité 4. Dans le mode de réalisation représenté, les cloisons 27 présentent une hauteur supérieure à 0,8 mm, de l'ordre de 1 mm.

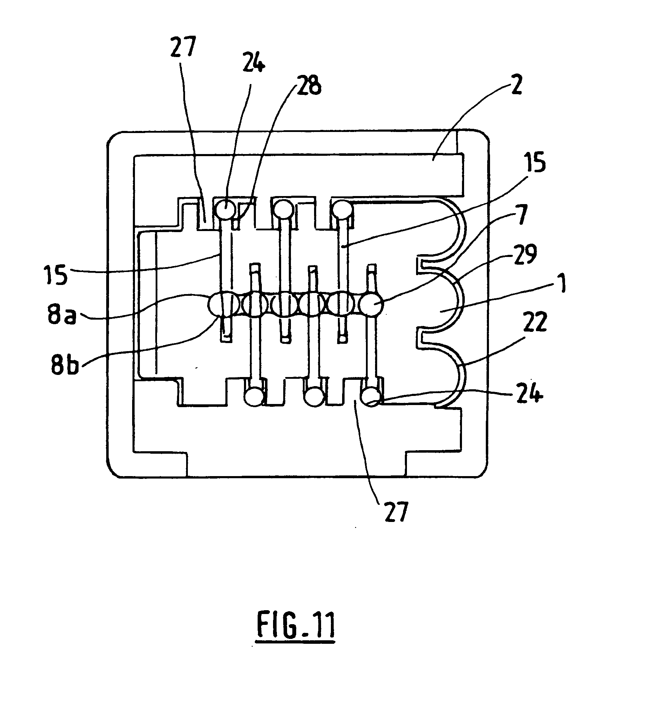

Comme représenté sur les figures 6 et 11, les dimensions des encoches 14 du connecteur mâle 1 sont agencées pour permettre l'insertion d'un doigt de contact 24 et d'une cloison d'isolation 27 à l'intérieure de ladite encoche 14. En effet, lorsque le connecteur mâle 1 vient s'insérer dans la cavité 4 du connecteur femelle 2, le doigt de contact 24 vient coopérer avec la griffe de contact 15 afin d'assurer la connexion et la cloison d'isolation 27 constitue avec la barrière 25 une séparation entre ladite encoche 14 et une encoche voisine 14.

Dans le mode de réalisation représenté sur la figure 11, la cloison d'isolation 27 est reçue dans un premier espace de réception s'étendant entre la griffe de contact 15 et un bord de l'encoche 14. La cloison d'isolation 27 coopère alors avec un bord de l'encoche 14 et constitue avec la barrière 25 une séparation isolante avec une encoche voisine.Par ailleurs, le doigt de contact 24 entre en contact, en vis-à-vis, avec la griffe de contact 15 et est reçue dans un second espace de réception s'étendant entre la griffe de contact 15 et le plan de la paroi 11a.

Dans un autre mode de réalisation non illustré, on pourra également prévoir que les doigts de contact 24 présentent une section cylindrique et soit reçus dans un premier espace de réception s'étendant entre la griffe de contact 15 et un bord de l'encoche 14. Dans ce cas, le contact entre le doigt 24 et la griffe 15 est latéral. Par ailleurs, la cloison d'isolation 27 est reçue dans un second espace de réception s'étendant entre la griffe de contact 15 et le plan de la paroi 11a.

On notera également que, dans le mode de réalisation représenté, une des parois 12b du boîtier 23 est équipée d'au moins un repère détrompeur 29, formé de trois cavités demi cylindriques. En outre, la paroi gauche 13b est équipée de moyens de butée permettant de maintenir le connecteur mâle dans la cavité.

Par ailleurs, le connecteur femelle 2 représenté est destiné à être connecté à un circuit imprimé. À cet effet, une partie des doigts de contact 24 émerge perpendiculairement sur le dessus du boîtier 23 sous forme de picots afin de permettre la connexion des doigts aux pistes du circuit imprimé. En outre, le boîtier 23 est muni de pattes de fixation 31 destinée à être introduites dans des orifices formés dans le circuit imprimé.

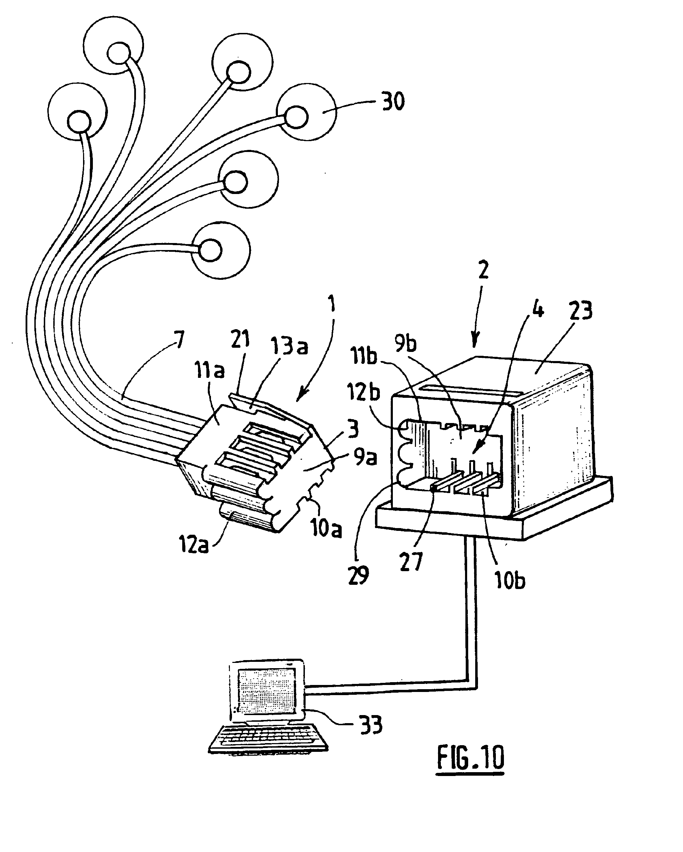

Le système médical, représenté sur la figure 10, comprend des électrodes 30 qui sont destinées à être disposées sur le corps d'un patient, un dispositif de connexion à des moyens de traitement des signaux 33 et des câbles 7. Le dispositif de connexion est composé des connecteurs, mâle 1 et femelle 2, selon l'invention. Les câbles 7 sont connectés, d'une part ,à une électrode 30 et, d'autre part, à une griffe de contact 15 du connecteur mâle 1.

Dans un mode de réalisation préféré de l'invention, les câbles 7 utilisés pour la liaison entre les électrodes 30 et le connecteur mâle 1 sont des câbles en nappe.

L'invention est décrite dans ce qui précède à titre d'exemple. Il est entendu que l'homme du métier est à même de réaliser différentes variantes de réalisation de l'invention sans pour autant sortir du cadre de l'invention.

On notera notamment que bien que les connecteurs représentés soient des connecteurs six câbles, il est également possible de réaliser, sur le même principe, des connecteurs pour 4, 5, 7, 8 câbles ou plus selon la configuration souhaitée.

## Revendications

1. Dispositif de connexion pour la transmission de signaux électriques comportant :
- un connecteur femelle (2) comprenant :
- un boîtier (23) comportant des parois (9b, 10b, 11 b, 12b, 13b) délimitant une cavité de réception (4) d'un connecteur mâle (1) ; et
- des premiers doigts de contacts (24), s'étendant parallèlement à une première paroi (10b, 11b) et destinés à coopérer avec des contacts (15) correspondants du connecteur mâle (1) ;
- un connecteur mâle (1) comprenant :
- un corps (3) en matériau diélectrique, destiné à être introduit dans le connecteur femelle (2), ledit corps (3) comportant :
- un logement (6) pourvu d'une ouverture (5) pour le passage de câbles (7) ;
- des faces extérieures (9a, 10a, 11a, 12a, 13a) destinées à venir en vis-à-vis des parois (9b, 10b, 11 b, 12b, 13b) délimitant la cavité (4) du connecteur femelle (2) ;
- des encoches (14) formées sur au moins une première face extérieure (10a, 11a) munies d'une lumière (16) débouchant dans ledit logement (6) ; et
- des griffes de contacts (15), insérées dans lesdites lumières (16), comportant une piste de contact (28) destinée à coopérer avec les doigts de contact (27) du connecteur femelle (2) et une portion auto dénudante (17) s'étendant dans le logement (6) ;
ledit dispositif de connexion étant caractérisé en ce le connecteur femelle (2) comporte des cloisons d'isolation (27) en matériau diélectrique faisant saillie de la première paroi (10b, 11b) vers l'intérieur de la cavité (4) et disposées entre les premiers doigts de contact (24) et en ce que chaque encoche (14) du connecteur mâle présente des dimensions adaptées pour recevoir un doigt de contact (24) et une des cloisons d'isolation (27) de manière à constituer une séparation entre ladite encoche (14) et une encoche (14) voisine.

2. Dispositif de connexion selon la revendication 1, **caractérisé en ce que** le connecteur femelle (2) comprend des seconds doigts de contact (24) s'étendant parallèlement à une seconde paroi (11b, 10b) opposée à la première paroi (10b, 11b) et des cloisons d'isolation (27) en matériau diélectrique faisant saillie de la seconde paroi (11b, 10b) vers l'intérieur de la cavité et disposées entre les seconds doigts de contact (24).

3. Dispositif de connexion selon la revendication 2, caractérisé les premiers et les seconds doigts de contact (24) sont disposés en quinconce les uns par rapport aux autres.

4. Dispositif de connexion l'une des revendications 1 à 3, **caractérisé en ce que** les doigts de contacts (24) sont éloignés d'une distance supérieure à 1 mm, de préférence supérieure ou égale à 1,4 mm.

5. Dispositif de connexion selon l'une des revendications 1 à 4, **caractérisé en ce que** les cloisons d'isolation (27) sont réalisées en polyamide.

6. Dispositif de connexion selon l'une des revendications 1 à 5, **caractérisé en ce que** les doigts de contacts (24) sont en laiton.

7. Dispositif de connexion selon l'une des revendications 1 à 6, **caractérisé en ce que** les doigts de contact (24) présentent une section cylindrique.

8. Dispositif de connexion selon l'une des revendications 1 à 7, **caractérisé en ce qu'**au moins un des parois (12b) du boîtier est équipé d'au moins un repère détrompeur (29).

9. Dispositif de connexion selon l'une des revendications 1 à 8, **caractérisé en ce qu'**au moins une des parois (13b) est équipée de moyens de butée permettant de maintenir le connecteur mâle (1) dans la cavité (4).

10. Dispositif de connexion selon l'une des revendications 1 à 9, **caractérisé en ce que** chaque encoche (14) présente, entre la lumière (16) et un bord de ladite encoche (14), un premier espace de réception d'une cloison d'isolation (27).

11. Dispositif de connexion selon l'une des revendications 1 à 10, **caractérisé en ce que** chaque encoche (14) présente, entre la griffe de contact (15) et le plan de la première paroi (10a, 11a), un second espace de réception d'un doigt de contact (24).

12. Dispositif de connexion selon l'une quelconque des revendications 1 à 11, **caractérisé en ce que** les encoches (16) présentent une profondeur supérieure à 0, 8 mm.

13. Dispositif de connexion selon l'une quelconque des revendications 1 à 12, **caractérisé en ce que** les encoches (16) présentent une largeur supérieure à 1 mm, de préférence supérieure à 1,4 mm.

14. Dispositif de connexion selon l'une des revendications 1 à 13, caractérisé en que des encoches (14) munies d'une lumière (16) débouchant dans le logement (6) sont formées sur une seconde face extérieure (11a, 10a), opposée à la première face extérieure (10a, 11a) ; des griffes de contacts (15) étant insérées dans lesdites lumières (16).

15. Dispositif de connexion selon la revendication 14, **caractérisé en ce que** les griffes de contact (15) insérées dans les lumières (16) de la première face extérieure (10a, 11a) et celles de la seconde face extérieure (11a, 10a) sont disposées en quinconce.

16. Dispositif de connexion selon l'une des revendications 1 à 15, **caractérisé en ce que** les pistes de contacts (28) d'une même face extérieure (10a, 11a) sont éloignés d'une distance supérieure à 1 mm, de préférence supérieure ou égale à 1,4 mm.

17. Dispositif de connexion selon l'une des revendications 1 à 16, **caractérisé en ce que** le corps (3) est réalisé en polycarbonate.

18. Dispositif de connexion selon l'une des revendications 1 à 17, **caractérisé en ce que** les griffes de contact (15) peuvent être en carbone.

19. Dispositif de connexion selon l'une des revendications 1 à 18, **caractérisé en ce que** la portion auto dénudante (17) présente une forme en W, s'étendant dans le plan longitudinal du connecteur (1) et dont les deux branches (18, 19) sont destinées à sectionner la gaine du câble (7).

20. Dispositif de connexion selon l'une des revendications 1 à 19, **caractérisé en ce qu'**au moins une des faces extérieures (12a) du corps est équipée d'au moins un repère détrompeur (22).

21. Dispositif de connexion selon l'une des revendications 1 à 20, **caractérisé en ce qu'**au moins une des faces extérieures (13a) est équipée de moyens de verrouillage comprenant une languette élastique (21) et une surface de butée destinée à coopérer avec une surface de butée du connecteur femelle (2).

22. Système médical d'acquisition de signaux électriques comprenant :
- des électrodes (30) destinées à être mise en contact avec le corps d'un patient ;
- un dispositif de connexion à des moyens de traitement des signaux électriques ;
- des câbles (7) connectés d'une part à une desdites électrodes (30) et d'autre part au dispositif de connexion ;
- ledit système médical étant **caractérisé en ce que** le dispositif de connexion est un dispositif de connexion selon l'une des revendications 1 à 21

## Claims

1. Connection device for the transmission of electric signals, including:
- a female connector (2) comprising:
o a casing (23) comprising walls (9b, 10b, 11b, 12b, 13b) delimiting a cavity (4) for receiving a male connector (1); and
o first contact fingers (24), extending parallel to a first wall (10b, 11b) and intended to cooperate with matching contacts (15) of the male connector (1);
- a male connector (1) comprising:
o a body (3) made of a dielectric material, intended to be introduced into the female connector (2), said body (3) comprising:
■ a housing (6) provided with an opening (5) for the passage of cables (7);
■ outer faces (9a, 10a, 11a, 12a, 13a) intended to be positioned opposite the walls (9b, 10b, 11b, 12b, 13b) delimiting the cavity (4) of the female connector (2);
■ notches (14) formed on at least a first outer face (10a, 11a) provided with an aperture (16) opening into said housing (6); and
o contact claws (15) inserted into said apertures (16) comprising a contact track (28) intended to cooperate with the contact fingers (27) of the female connector (2) and a self-baring portion (17) extending in the housing (6);
said connection device being **characterised in that** the female connector (2) comprises isolation partitions (27) made of a dielectric material projecting from the first wall (10b, 11b) towards the inside of the cavity (4) and positioned between the first contact fingers (24), and **in that** each notch (14) of the male connector has dimensions suitable for receiving a contact finger (24) and one of the isolation partitions (27) so as to constitute a partition between said notch (14) and an adjacent notch (14).

2. Connection device according to claim 1, **characterised in that** the female connector (2) comprises second contact fingers (24) extending parallel to a second wall (11b, 10b) opposite to the first wall (10b, 11b) and isolation partitions (27) made of a dielectric material projecting from the second wall (11b, 10b) towards the inside of the cavity and positioned between the second contact fingers (24).

3. Connection device according to claim 2, **characterised in that** the first and second contact fingers (24) are positioned in staggered rows with respect to one another.

4. Connection device according to one of claims 1 to 3, **characterised in that** the contact fingers (24) are separated by a distance greater than 1 mm, preferably greater than or equal to 1.4 mm.

5. Connection device according to one of claims 1 to 4, **characterised in that** the isolation partitions (27) are made of polyamide.

6. Connection device according to one of claims 1 to 5, **characterised in that** the contact fingers (24) are made of brass.

7. Connection device according to one of claims 1 to 6, **characterised in that** the contact fingers (24) have a cylindrical cross-section.

8. Connection device according to one of claims 1 to 7, **characterised in that** at least one of the walls (12b) of the case is provided with at least one polarising slot (29).

9. Connection device according to one of claims 1 to 8, **characterised in that** at least one of the walls (13b) is provided with abutting means for holding the male connector (1) in the cavity (4).

10. Connection device according to one of claims 1 to 9, **characterised in that** each notch (14) has, between the aperture (16) and an edge of said notch (14) a first space for receiving an isolation partition (27).

11. Connection device according to one of claims 1 to 10, **characterised in that** each notch (14) has, between the contact claw (15) and the plane of the first wall (10a, 11a), a second space for receiving a contact finger (24).

12. Connection device according to any one of claims 1 to 11, **characterised in that** the notches (14) have a depth greater than 0.8 mm.

13. Connection device according to one of claims 1 to 12, **characterised in that** the notches (14) have a width greater than 1 mm, preferably greater than 1.4 mm.

14. Connection device according to one of claims 1 to 13, **characterised in that** notches (14) provided with an aperture (16) opening in the housing (6) are formed on a second outer face (11a, 10a) opposite to the first outer face (10a, 11a); contact claws (15) being inserted into said apertures (16).

15. Connection device according to claim 14, **characterised in that** the contact claws (15) inserted into the apertures (16) in the first outer face (10a, 11a) and those in the second outer face (11a, 10a) are positioned in a staggered rows.

16. Connection device according to one of claims 1 to 15, **characterised in that** the contact tracks (28) of the same outer face (10a, 11a) are separated by a distance greater than 1 mm, preferably greater than or equal to 1.4 mm.

17. Connection device according to one of claims 1 to 16, **characterised in that** the body (3) is made of polycarbonate.

18. Connection device according to one of claims 1 to 17, **characterised in that** the contact claws (15) may be made of carbon.

19. Connection device according to one of claims 1 to 18, **characterised in that** the self-baring portion (17) is W-shaped, extending in the longitudinal plane of the connector (1), and the two arms (18, 19) of which are intended to cut the sheath of the cable (7).

20. Connection device according to one of claims 1 to 19, **characterised in that** at least one of the outer faces (12a) of the body is provided with at least one polarising slot (22).

21. Connection device according to one of claims 1 to 20, **characterised in that** at least one of the outer faces (13a) is provided with locking means comprising a resilient lug (21) and an abutting surface intended to cooperate with an abutting surface of the female connector (2).

22. Medical system for acquisition of electric signals, including:
- electrodes (30) intended to be put in contact with the body of a patient;
- a connection device with electric signals processing means;
- cables (7) connected on the one hand to one of said electrodes (30) and on the other hand to the connection device;
- said medical system being **characterised in that** the connection device is a connection device according to one of claims 1 to 21.

## Patentansprüche

1. Anschlussvorrichtung zur Übertragung von elektrischen Signalen umfassend:
- eine Buchse (2) umfassend:
- ein Gehäuse (23) umfassend Wände (9b, 10b, 11b, 12b, 13b), die einen Aufnahmehohlraum (4) eines Steckers (1) begrenzen; und
- erste Kontaktfinger (24), die sich parallel zu einer ersten Wand (10b, 11b) erstrecken und dazu bestimmt sind, mit den entsprechenden Kontakten (15) des Steckers (1) zu kooperieren;
- einen Stecker (1) umfassend:
- einen Körper (3) aus dielektrischem Material, dazu bestimmt, in die Buchse (2) eingeführt zu werden, wobei besagter Körper (3) Folgende umfasst:
- ein mit einer Öffnung (5) für den Durchgang der Kabel (7) versehenes Gehäuse (6);
- Außenseiten (9a, 10a, 11a, 12a, 13a) dazu bestimmt die gegenüberliegende Seite der Wände zu bilden (9b, 10b, 11b, 12b, 13b), die den Hohlraum (4) der Buchse (2) begrenzen;
- an mindestens einer der Außenseiten (10a, 11a) gebildete Aussparungen (14) mit einer Öffnung (16) versehen, die in besagtes Gehäuse (6) mündet; und
- in besagte Öffnungen (16) eingesetzte Kontaktklauen (15) umfassend eine Kontaktspur (28) dazu bestimmt, mit den Kontaktfingern (27) der Buchse (2) zu kooperieren und einen Selbst- Abisolierbereich (17), der sich in das Gehäuse erstreckt (6);
wobei besagte Anschlussvorrichtung **dadurch gekennzeichnet ist, dass** die Buchse (2) Isolierwände (27) aus dielektrischem Material umfasst, die über die erste Wand (10b, 11b) hinaus ins Innere des Hohlraums (4) ragen und zwischen den ersten Kontaktfingern (24) angebracht sind, und wobei jede Aussparung (14) des Steckers eine zur Aufnahme eines Kontaktfingers (24) und einer der Isolierwände (27) geeignete Größe aufweist, so dass eine Abtrennung zwischen besagter Aussparung (14) und einer benachbarten Aussparung gebildet wird (14).

2. Anschlussvorrichtung nach Anspruch 1, **dadurch gekennzeichnet, dass** die Buchse (2) zweite Kontaktfinger (24) umfasst, die sich parallel zu einer zweiten Wand (11b, 10b) gegenüber der ersten Wand (10b, 11b) erstrecken, und Isolierwände (27) aus dielektrischem Material, die über die zweite Wand (11b, 10b) hinaus ins Innere des Hohlraums ragen und zwischen den zweiten Kontaktfingern(24) angebracht sind.

3. Anschlussvorrichtung nach Anspruch 2, **dadurch gekennzeichnet, dass** die ersten und zweiten Kontaktfinger (24) gegeneinander versetzt angebracht sind.

4. Anschlussvorrichtung nach einem beliebigen der Ansprüche 1 bis 3, **dadurch gekennzeichnet, dass** die Kontaktfinger (24) mehr als 1 mm entfernt sind, vorzugsweise größer gleich 1,4 mm.

5. Anschlussvorrichtung nach einem beliebigen der Ansprüche 1 bis 4, **dadurch gekennzeichnet, dass** die Isolierwände (27) aus Polyamid hergestellt sind.

6. Anschlussvorrichtung nach einem beliebigen der Ansprüche 1 bis 5, **dadurch gekennzeichnet, dass** die Kontaktfinger (24) aus Messing hergestellt sind.

7. Verbindungsvorrichtung nach einem beliebigen der Ansprüche 1 bis 6, **dadurch gekennzeichnet, dass** die Kontaktfinger (24) einen zylindrischen Querschnitt haben.

8. Anschlussvorrichtung nach einem beliebigen der Ansprüche 1 bis 7, **dadurch gekennzeichnet, dass** mindestens eine der Wände (12b) des Gehäuses mit mindestens einem indizierten Platz (29) ausgestattet ist.

9. Anschlussvorrichtung nach einem beliebigen der Ansprüche 1 bis 8, **dadurch gekennzeichnet, dass** mindestens eine der Wände (13b) mit einem Anschlag zum Halten des Steckers (1) im Hohlraum (4) ausgestattet ist.

10. Anschlussvorrichtung nach einem beliebigen der Ansprüche 1 bis 9, **dadurch gekennzeichnet, dass** jede Aussparung (14) zwischen der Öffnung (16) und einer Kante der besagten Aussparung (14) einen ersten Aufnahmeraum einer Isolierwand (27) aufweist.

11. Anschlussvorrichtung nach einem beliebigen der Ansprüche 1 bis 10, **dadurch gekennzeichnet, dass** jede Aussparung (14) zwischen der Kontaktklaue (15) und der Ebene der ersten Wand (10a, 11a) einen zweiten Aufnahmeraum für einen Kontaktfinger (24) hat.

12. Anschlussvorrichtung nach einem beliebigen der Ansprüche 1 bis 11, **dadurch gekennzeichnet, dass** die Tiefe der Aussparungen (16) größer als 0,8 mm ist.

13. Anschlussvorrichtung nach einem beliebigen der Ansprüche 1 bis 12, **dadurch gekennzeichnet, dass** die Weite der Aussparungen (16) größer als 1 mm ist, vorzugsweise größer als 1,4 mm.

14. Anschlussvorrichtung nach einem beliebigen der Ansprüche 1 bis 13, **dadurch gekennzeichnet, dass** die mit einer in das Gehäuse (6) mündenden Öffnung (16) versehenen Aussparungen (14) an einer zweiten, der ersten Außenseite (10a, 11a) gegenüberliegenden Außenseite (11a, 10a) gebildet sind; wobei Kontaktklauen (15) in besagte Öffnungen (16) eingesetzt sind.

15. Anschlussvorrichtung nach Anspruch 14, **dadurch gekennzeichnet, dass** die in die Öffnungen (16) in der ersten Außenseite (10a, 11a) und in der zweiten Außenseite (11a, 10a) eingesetzten Kontaktklauen (15) versetzt angebracht sind.

16. Anschlussvorrichtung nach einem beliebigen der Ansprüche 1 bis 15, **dadurch gekennzeichnet, dass** die Kontaktpisten (28) einer gleichen Außenseite (10a, 11a) mehr als 1 mm voneinander entfernt sind, vorzugsweise größer gleich 1,4 mm.

17. Anschlussvorrichtung nach einem beliebigen der Ansprüche 1 bis 16, **dadurch gekennzeichnet, dass** der Körper (3) aus Polycarbonat hergestellt ist.

18. Anschlussvorrichtung nach einem beliebigen der Ansprüche 1 bis 17, **dadurch gekennzeichnet, dass** die Kontaktklauen (15) aus Kohlenstoff hergestellt sein können.

19. Anschlussvorrichtung nach einem beliebigen der Ansprüche 1 bis 18, **dadurch gekennzeichnet, dass** der Selbst-Abisolierbereich (17) eine W-Form aufweist, die sich in der Längsebene des Steckers (1) erstreckt, und deren zwei Äste (18, 19) dazu bestimmt sind, die Kabelummantelung zu durchschneiden (7).

20. Anschlussvorrichtung nach einem beliebigen der Ansprüche 1 bis 19, **dadurch gekennzeichnet, dass** mindestens eine der Außenseiten (12a) des Körpers mit mindestens einem indizierten Platz (22) versehen ist.

21. Anschlussvorrichtung nach einem beliebigen der Ansprüche 1 bis 20, **dadurch gekennzeichnet, dass** mindestens eine der Außenseiten (13a) mit Sperren ausgestattet ist, umfassend eine elastische Lasche (21) und eine Anschlagsfläche, dazu bestimmt, mit der Anschlagsfläche der Buchse (2)zu kooperieren.

22. Medizinisches System zur Erfassung elektrischer Signale umfassend:
- Elektroden (30) zum in Kontaktbringen mit dem Körper eines Patienten bestimmt;
- eine Anschlussvorrichtung zu Mitteln zur Verarbeitung elektrischer Signale;
- Kabel (7) zum einen an besagte Elektroden (30) und zum anderen an die Anschlussvorrichtung angeschlossen;
- wobei besagtes medizinisches System **dadurch gekennzeichnet ist, dass** die Anschluss-vorrichtung eine Anschlussvorrichtung nach einem beliebigen der Ansprüche 1 bis 21 ist.
